# EUROPEAN PATENT APPLICATION

(11) **EP 4 700 043 A1**
(43) Date of publication of application: **25.02.2026**
(21) Application number: 24792695.9
(22) Date of filing: 17.04.2024
(51) Int. Cl.: C07K 14/435, A01N 37/46, A01N 63/16, A01P 7/04, C12N 15/12

(54) **INSECTICIDAL PEPTIDE DERIVED FROM SCORPION VENOM**

(30) Priority: 18.04.2023 JP 2023068072
(71) Applicant: Kyoto University, Kyoto-shi, Kyoto 606-8501 (JP); Nippon Soda Co., Ltd., Tokyo 100-7010 (JP)
(72) Inventor: MIYASHITA, Masahiro, Kyoto-shi, Kyoto 606-8501 (JP); MITANI, Naoya, Kyoto-shi, Kyoto 606-8501 (JP); IWAMOTO, Fuki, Kyoto-shi, Kyoto 606-8501 (JP); NAKAGAWA, Yoshiaki, Kyoto-shi, Kyoto 606-8501 (JP); MIYAGAWA, Hisashi, Kyoto-shi, Kyoto 606-8501 (JP)
(74) Representative: Mewburn Ellis LLP
(86) International application number: PCT/JP2024/015265
(87) International publication number: WO 2024/219423

(57) **Abstract**

Provided are: a peptide having insecticidal activity and containing an amino acid sequence represented by SEQ ID NO: 2, SEQ ID NO: 5, or SEQ ID NO: 7, or an amino acid sequence containing one to several amino acid mutations in SEQ ID NO: 2, SEQ ID NO: 5, or SEQ ID NO: 7; an insecticidal composition containing the peptide; and a pest control method using the peptide.

## Description

### TECHNICAL FIELD

The present invention relates to a novel peptide having an insecticidal activity and being derived from scorpion venom, an insecticidal composition containing the peptide, and a method for controlling insect pests including applying the peptide.

### BACKGROUND ART

Scorpions are known to use venom to capture insects as food and to defend themselves against natural enemies. The main bioactive components of scorpion venom are peptides, and the venom of one species of scorpion contains more than 100 types of peptide components with a wide range of molecular weights, from about 500 Da to about 9000 Da. The types and mass distribution of bioactive peptides contained in the venom of scorpions vary depending on the species of the scorpions.

Major peptides in scorpion venom are a neurotoxins that can instantly stop the movement of prey by acting on ion channels. Scorpion venom contains not only peptides that act on ion channels, but also peptides that exhibit antimicrobial activity. All scorpion venom peptides that act on ion channels are known to be cross-linked by disulfide bonds. Scorpion venom peptides are classified based on their action on ion channels or other characteristic actions, or on their disulfide bond cross-linking patterns or other characteristic structures. Some scorpion venom peptides exhibit high selectivity of action against mammals or insects.

Since scorpion venom contains diverse components, the scorpion venom has been studied as a rich source of bioactive molecules useful to develop novel agrochemicals or medicines. Various neurotoxic, antimicrobial, or insecticidal peptides derived from scorpion venoms have been isolated and identified to date. For example, the present inventors' group previously identified the insecticidal peptides LaIT1 (Non-Patent Document 1) and LaIT2 (Non-Patent Document 2) from the venom of the scorpion *Liocheles australasiae.*

However, details of only around 1,000 types of scorpion venom peptides have been clarified to date. Currently, the presence of 2,400 or more types of scorpions have been confirmed, and if each venom thereof contains 100 types of bioactive peptide components, a total of 240,000 types of scorpion venom peptides would exist. Namely, scorpion venom peptides that have been identified to date account for less than 1% of the total. Therefore, it is expected that even more useful scorpion venom peptides will be found.

### Citation List

### Non Patent Document

Non Patent Document 1: Toxicon, 50, 861 (2007)
Non Patent Document 2: Biosci. Biotechnol. Biochem., 73, 2769 (2009)

### SUMMARY OF INVENTION

### Technical Problem

An object of the present invention is to obtain a novel peptide having an insecticidal activity and being derived from scorpion venom.

### Solution to Problem

As a result of intensive research under the above-mentioned circumstances, the present inventors surprisingly obtain a novel peptide having an insecticidal activity from peptides classified into the κ family (called κ-KTx peptides) among KTx peptides (peptides classified in terms of having structures similar to those of peptides that act on potassium ion channels) derived from the venom of the *L. australasiae* scorpion. There have been no reports of κ-KTx peptides having an insecticidal activity to date. Even more surprisingly, a peptide having an improved insecticidal activity has been obtained by modifying an amino acid sequence of the κ-KTx peptide obtained from the venom of the *L. australasiae* scorpion (hereinafter, referred to as "La-κKTx"). Thus, the present invention has been completed.

The present invention encompasses the following aspects.
[1] A peptide having an insecticidal activity, the peptide containing an amino acid sequence of SEQ ID NO: 2, SEQ ID NO: 5, or SEQ ID NO: 7, or an amino acid sequence having one to several amino acid mutations in the amino acid sequence of SEQ ID NO: 2, SEQ ID NO: 5, or SEQ ID NO: 7.
[2] The peptide according to [1], wherein an amino acid residue corresponding to a position 10 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is a basic amino acid.
[3] The peptide according to [1] or [2], wherein an amino acid residue corresponding to a position 6 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is an acidic amino acid.
[4] The peptide according to any one of [1] to [3], wherein an amino acid residue corresponding to a position 11 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is a basic amino acid.
[5] The peptide according to any one of [1] to [4], wherein at least one amino acid residue corresponding to at least one position of positions 13 or later in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is substituted with another amino acid.
[6] The peptide according to [5], wherein the at least one amino acid residue corresponding to at least one position of positions 13 or later is at least one amino acid residue selected from the group consisting of amino acid residues corresponding to positions 17, 19, 20, 23 and 24.
[7] The peptide according to any one of [1] to [6], wherein a C-terminus is amidated.
[8] An insecticidal composition containing the peptide of any one of [1] to [7].
[9] The insecticidal composition according to [8], wherein the insecticidal composition is in a form selected from the group consisting of oil agents, emulsions, liquid agents, flowable agents, water-soluble agents, wettable powders, water dispersible granules, powders, granules, powdered granules, microcapsules and aerosols.
[10] A method for controlling insect pests, including applying the peptide of any one of [1] to [7] to the insect pests or a habitat of the insect pests.
[11] A nucleic acid encoding the peptide of any one of [1]~[6].

### Advantageous Effects of Invention

The present invention provides a novel insecticidal peptide derived from scorpion venom. The present invention provides an insecticidal peptide with high selectivity that is not toxic to mammals such as mice and exhibits insecticidal action on insects specifically. Such a peptide is useful particularly in the field of agrochemicals.

### BRIEF DESCRIPTION OF DRAWINGS

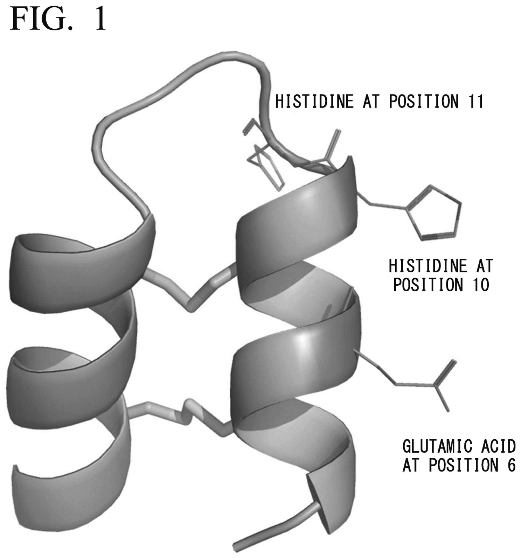

[FIG. 1] Fig. 1 indicates a 3D structure of La-κKTx2. This figure indicates the presence of a histidine residue at position 10, a histidine residue at position 11, and a glutamic acid residue at position 6 on one of two α-helix structures cross-linked by two pairs of disulfide bond.

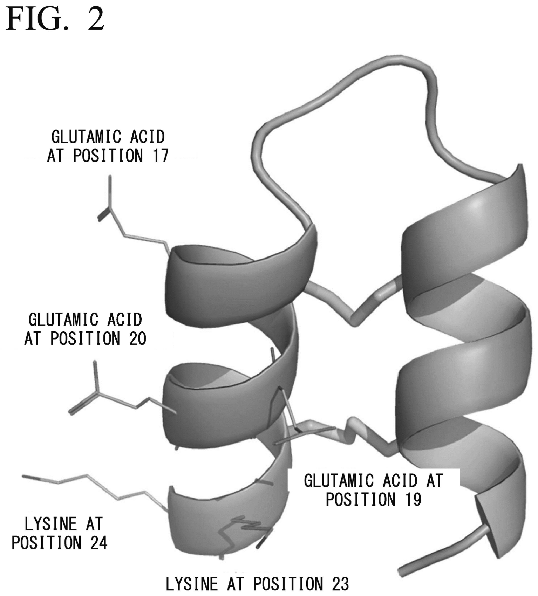

[FIG. 2] Fig. 2 indicates a 3D structure of La-κKTx2. This figure indicates the presence of a glutamic acid residue at position 17, a glutamic acid residue at position 19, a glutamic acid residue at position 20, a lysine residue at position 23 and a lysine residue at position 24 on one of two α-helix structures cross-linked by two pairs of disulfide bond. All of these residues increased the insecticidal activity when substituted with alanine (Example 6).

### DESCRIPTION OF EMBODIMENTS

### 1. Insecticidal peptide

Bioactive peptides were conventionally searched by an activity-based search method in which fractionation by high performance liquid chromatography (HPLC) and activity evaluation were repeated until a single active component was obtained, followed by carrying out isolation and purification to determine the structure thereof. However, it was difficult to obtain an active component contained in a low amount in venom by such an approach. Therefore, in the present application, a structure-based search method was used in which the structures of all components contained in the venom were determined based on the transcriptomic and mass spectrometric information of the scorpion venom gland, followed by synthesizing peptides predicted to have an activity and then evaluating the activity thereof. Specifically, total mRNA was extracted from the telson of the scorpion *Liocheles australasiae* belonging to the family Honnuridae, a cDNA library was prepared by reverse transcription, the nucleotide sequence was determined by a nextgeneration sequencer, the amino acid sequence was determined based on the nucleotide sequence, and bioactive peptide candidates were obtained by structural comparison with known scorpion venom bioactive peptides. Next, the bioactive peptide candidates were chemically synthesized, and the insecticidal activity thereof was evaluated.

Thus, five peptides, La-κKTx1, La-κKTx2, La-κKTx3, La-κKTx4, and La-κKTx5, whose sequences are similar to known κ-KTx peptides, were selected as bioactive peptide candidates in the present application. Their amino acid sequences are shown in Table 1 (SEQ ID NOs: 1 to 5, respectively). KTx peptides are classified into seven families (α, β, γ, κ, δ, λ, and ε) based on their structures. The x-KTx peptides are peptides that have a cystine-stabilized (CS) α/α motif in which two α helices are fixed by two pairs of disulfide bond. The La-κKTx1 has disulfide bonds between cysteine residues at positions 4 and 22 and between cysteine residues at positions 8 and 18 in SEQ ID NO: 1, the La-κKTx2 has disulfide bonds between cysteine residues at positions 4 and 22 and between cysteine residues at positions 8 and 18 in SEQ ID NO: 2, the La-xKTx3 has disulfide bonds between cysteine residues at positions 6 and 21 and between cysteine residues at positions 10 and 17 in SEQ ID NO: 3, the La-κKTx4 has disulfide bonds between cysteine residues at positions 6 and 24 and between cysteine residues at positions 10 and 20 in SEQ ID NO: 4, and the La-κKTx5 has disulfide bonds between cysteine residues at positions 2 and 19 and between cysteine residues at positions 6 and 15 in SEQ ID NO: 5. The presence of components with the same masses as those of the above-mentioned bioactive peptide candidates in the venom extracted from the *L. australasiae* scorpions was confirmed from the mass spectrometry information thereof, and the peptides were chemically synthesized.

As a result of evaluating the insecticidal activity of the above-mentioned five peptide candidates, it was confirmed that two peptides, the La-κKTx2 and the La-κKTxS, exhibited insecticidal activity. Therefore, in one aspect of the present application, a peptide having an insecticidal activity and including an amino acid sequence of SEQ ID NO: 2 or SEQ ID NO: 5 is provided. In an additional aspect, a peptide consisting of the amino acid sequence of SEQ ID NO: 2 or SEQ ID NO: 5 is provided.

Furthermore, it has been surprisingly found in the present application that the presence of a basic amino acid at position 10 in the amino acid sequence of the La-κKTx2 (SEQ ID NO: 2) is important in terms of the insecticidal activity thereof. Furthermore, the insecticidal activity was improved, namely, a peptide having an insecticidal activity was obtained, by substituting glutamine at position 10 in the amino acid sequence of the La-κKTx1 (SEQ ID NO: 1), which did not exhibit insecticidal activity, with a basic amino acid (Example 4). Accordingly, in another aspect of the present application, a peptide having an insecticidal activity in which glutamine at position 10 in the amino acid sequence of the La-κKTx1 (SEQ ID NO: 1) is substituted with a basic amino acid is provided. In the present specification, examples of the basic amino acid include histidine, lysine, and arginine.

As an example of the peptide in which glutamine at position 10 in the amino acid sequence of the La-κKTx1 (SEQ ID NO: 1) is substituted with a basic amino acid, a peptide in which glutamine at position 10 in the amino acid sequence of the La-κKTx1 is substituted with histidine [referred to as La-κKTx1(Q10H)] can be mentioned. The amino acid sequence of the La-κKTx1(Q1OH) is shown as SEQ ID NO: 7. Thus, in an additional aspect of the present application, a peptide having an insecticidal activity and including the amino acid sequence of SEQ ID NO: 7, preferably consisting of the amino acid sequence of SEQ ID NO: 7 is provided. In another aspect, a peptide having an insecticidal activity and including an amino acid sequence in which glutamine at position 10 in the amino acid sequence of SEQ ID NO: 1 is substituted with lysine or arginine, preferably consisting of the amino acid sequence in which glutamine at position 10 in the amino acid sequence of SEQ ID NO: 1 is substituted with lysine or arginine is provided.

Furthermore, in the present application, it was found that the presence of an acidic amino acid at position 6 in the amino acid sequence of the La-xKTx2 (SEQ ID NO: 2) is involved to some extent in its insecticidal activity (Example 4). Similarly, it is believed that the presence of an acidic amino acid at position 6 in the La- 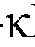 KTx1(Q10H) (SEQ ID NO: 7), which has an amino acid sequence similar to that of the La-κKTx2, is also involved to some extent in its insecticidal activity. In the present specification, examples of the acidic amino acid include glutamic acid and aspartic acid.

Furthermore, in the present application, it was found that the presence of a basic amino acid at position 11 in the amino acid sequence of the La-κKTx2 (SEQ ID NO: 2) is involved in its insecticidal activity (Example 6). Similarly, it is believed that the presence of a basic amino acid at position 11 in the La- 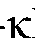 KTx1(Q10H) (SEQ ID NO: 7), which has an amino acid sequence similar to that of the La-κKTx2, is also involved in its insecticidal activity.

Any of the above-mentioned peptides may contain amino acid mutations so long as the peptides have insecticidal activity. Thus, in an additional aspect of the present application, a mutant of a peptide including the amino acid sequence of SEQ ID NO: 2, SEQ ID NO: 5, or SEQ ID NO: 7, with one to several amino acid mutations in the amino acid sequence is provided. In another aspect, a mutant of a peptide including the amino acid sequence of SEQ ID NO: 2, SEQ ID NO: 5, or SEQ ID NO: 7, with at least 60%, 65%, 70%, 75%, 80%, 85%, or 90%, preferably at least 95%, more preferably at least 96%, 97%, 98%, or 99% sequence identity to the amino acid sequence of SEQ ID NO: 2, SEQ ID NO: 5, or SEQ ID NO: 7 is provided. All of such peptide mutants retain insecticidal activity.

In the present specification, the term "several" means about 2 to 9, and may be, for example, 3, 4, 5, 6, 7, or 8. Thus, in an additional aspect of the present application, a peptide mutant including an amino acid sequence containing one to several, for example, 1 to 9, preferably 1 to 8, 1 to 7, 1 to 6, or 1 to 5, and more preferably 1 to 4, 1 to 3, or 1 or 2 amino acid mutations in the amino acid sequence of SEQ ID NO: 2, SEQ ID NO: 5, or SEQ ID NO: 7 is provided.

In the present specification, the term "amino acid mutation" encompasses substitution, deletion, insertion, and addition of amino acids. Examples of the substitution of amino acids include non-conservative substitution and conservative substitution. The conservative substitution of amino acids is a substitution between amino acids belonging to groups having the same or similar properties, and is well known in the art. Examples thereof include substitution between basic amino acids: arginine, lysine, and histidine, substitution between acidic amino acids: aspartic acid and glutamic acid, substitution between neutral amino acids: alanine, glycine, leucine, isoleucine, valine, serine, threonine, cysteine, methionine, asparagine, glutamine, proline, phenylalanine, tyrosine, and tryptophan, for example, substitution between hydrophobic/neutral amino acids: glycine, alanine, leucine, isoleucine, valine, proline, tryptophan, phenylalanine, and methionine, and substitution between hydrophilic/neutral amino acids: serine, threonine, cysteine, tyrosine, asparagine, and glutamine, but the conservative substitution is not limited thereto. In the present specification, the term "neutral amino acid" encompasses amino acids other than basic amino acids and acidic amino acids.

Since the presence of a basic amino acid at position 10 in the amino acid sequence of the La-κKTx2 or La-κKTx1(Q10H) is important in terms of insecticidal activity, as described above, the above-mentioned peptide mutant preferably contains a sequence in which the amino acid residue corresponding to the position 10 in the amino acid sequence of SEQ ID NO: 2 or SEQ ID NO: 7 is a basic amino acid. For example, the above-mentioned peptide mutant may contain an amino acid sequence in which histidine at the position corresponding to the position 10 in the amino acid sequence of SEQ ID NO: 2 or SEQ ID NO: 7 is substituted with another basic amino acid. For example, the histidine at the position corresponding to the position 10 in the amino acid sequence of SEQ ID NO: 2 or SEQ ID NO: 7 may not be substituted or may be substituted with another basic amino acid. For example, the above-mentioned peptide mutant may contain an amino acid sequence in which one to several amino acids at positions other than the position corresponding to the position 10 in the amino acid sequence of SEQ ID NO: 2 or SEQ ID NO: 7 are substituted. For example, the above-mentioned peptide mutant may contain an amino acid sequence in which histidine at the position corresponding to the position 10 in the amino acid sequence of SEQ ID NO: 2 or SEQ ID NO: 7 is unsubstituted or substituted with another basic amino acid, and one to several amino acids at positions other than the position corresponding to the position 10 are substituted.

In the present specification, with regard to the peptide mutant, the term "position corresponding to" or "amino acid residue corresponding to" a specific amino acid position in a specific amino acid sequence means a position in the mutated amino acid sequence that corresponds to a specific position in the amino acid sequence before the mutation, or the amino acid residue at the position. For example, if the mutation is a deletion, insertion, or addition, the amino acid position in the mutated amino acid sequence may be shifted compared to the amino acid sequence before the mutation, and in such a case, the term means a position in the mutated amino acid sequence that corresponds to the amino acid position before the mutation, or the amino acid residue at the position. For example, in a case in which the mutation is a substitution, the amino acid positions of the amino acid sequence before the mutation and the amino acid sequence after the mutation are identical to each other.

Furthermore, since the acidic amino acid at position 6 in the amino acid sequence of the La-κKTx2 or La-κKTx1(Q10H) is involved in the insecticidal activity as described above, the peptide mutant more preferably includes a sequence in which the amino acid residue corresponding to position 6 in the amino acid sequence of SEQ ID NO: 2 or SEQ ID NO: 7 is an acidic amino acid. For example, a glutamic acid at the position corresponding to position 6 in the amino acid sequence of SEQ ID NO: 2 or SEQ ID NO: 7 may be unsubstituted or substituted with another acidic amino acid (such as aspartic acid). More preferably, the peptide mutant may include a basic amino acid residue at the position corresponding to position 10 in the amino acid sequence of SEQ ID NO: 2 or SEQ ID NO: 7 and may include an acidic amino acid at the position corresponding to position 6 therein. For example, the above-mentioned peptide mutant may include an amino acid sequence in which a histidine at the position corresponding to position 10 in the amino acid sequence of SEQ ID NO: 2 or SEQ ID NO: 7 is unsubstituted or substituted with another basic amino acid, a glutamic acid at the position corresponding to position 6 therein is unsubstituted or substituted with another acidic amino acid, and one to several amino acids at positions other than those corresponding to positions 6 and 10 are substituted.

Furthermore, since the basic amino acid at position 11 in the amino acid sequence of the La-κKTx2 or La-κKTx1(Q10H) is involved in the insecticidal activity, as described above, the peptide mutant more preferably includes a sequence in which the amino acid residue corresponding to position 11 in the amino acid sequence of SEQ ID NO: 2 or SEQ ID NO: 7 is a basic amino acid. For example, a histidine at the position corresponding to position 11 in the amino acid sequence of SEQ ID NO: 2 or SEQ ID NO: 7 may be unsubstituted or substituted with another basic amino acid. More preferably, the above-mentioned peptide mutant includes a basic amino acid residue at the position corresponding to position 10 in the amino acid sequence of SEQ ID NO: 2 or SEQ ID NO: 7, and may also include a basic amino acid at the position corresponding to position 11. For example, the above-mentioned peptide mutant may include an amino acid sequence in which a histidine at the position corresponding to position 10 in the amino acid sequence of SEQ ID NO: 2 or SEQ ID NO: 7 is unsubstituted or substituted with another basic amino acid, a histidine at the position corresponding to position 11 is unsubstituted or substituted with another basic amino acid, and one to several amino acids at positions other than positions corresponding to positions 10 and 11 are substituted. As another preferred example, the peptide mutant may contain an acidic amino acid at the position corresponding to position 6 in the amino acid sequence of SEQ ID NO: 2 or SEQ ID NO: 7 and a basic amino acid residue at the position corresponding to position 11. For example, the above-mentioned peptide mutant may contain an amino acid sequence in which a glutamic acid at the position corresponding to position 6 in the amino acid sequence of SEQ ID NO: 2 or SEQ ID NO: 7 is unsubstituted or substituted with another acidic amino acid, a histidine at the position corresponding to position 11 is unsubstituted or substituted with another basic amino acid, and one to several amino acids at positions other than positions corresponding to positions 6 and 11 are substituted. More preferably, the above-mentioned peptide mutant may contain a basic amino acid residue at a position corresponding to position 10 in the amino acid sequence of SEQ ID NO: 2 or SEQ ID NO: 7, an acidic amino acid at a position corresponding to position 6, and a basic amino acid at a position corresponding to position 11. For example, the above-mentioned peptide mutant may contain an amino acid sequence in which a histidine at the position corresponding to position 10 in the amino acid sequence of SEQ ID NO: 2 or SEQ ID NO: 7 is unsubstituted or substituted with another basic amino acid, a glutamic acid at the position corresponding to position 6 is unsubstituted or substituted with another acidic amino acid, a histidine at the position corresponding to position 11 is unsubstituted or substituted with another basic amino acid, and one to several amino acids at positions other than those corresponding to positions 6, 10, and 11 are substituted.

Furthermore, it has been found in the present application that the insecticidal activity is enhanced when amino acids at positions 13 or later in the amino acid sequence of the La-κKTx2 (SEQ ID NO: 2) are substituted with any of other amino acids (Example 6). Similarly, it is believed that the La-κKTx1(Q10H) (SEQ ID NO: 7), which has an amino acid sequence similar to that of the La-κKTx2, also exhibits an enhanced insecticidal activity when amino acids at positions 13 or later are substituted with any of other amino acids. Preferable examples of the positions of the amino acids at positions 13 or later include positions 17, 19, 20, 23, and 24, and one or more of these positions may be substituted.

Therefore, the above-mentioned peptide mutant may include an amino acid sequence in which an amino acid at a position corresponding to position 13 or later in the amino acid sequence of SEQ ID NO: 2 or SEQ ID NO: 7 is substituted with any of other amino acids. For example, the above-mentioned peptide mutant may include an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of other amino acids. Such a substitution may be a conservative substitution or a non-conservative substitution. The substitution is preferably the non-conservative substitution, and examples thereof include a substitution of a basic amino acid with an acidic amino acid or a neutral amino acid, and a substitution of an acidic amino acid with a basic amino acid or a neutral amino acid. For example, the above-mentioned peptide mutant may include an amino acid sequence in which one or more basic amino acid residues and/or acidic amino acid residues selected from the group consisting of amino acid residues corresponding to positions 17, 19, 20, 23, and 24 in the amino acid sequence of SEQ ID NO: 2 or SEQ ID NO: 7 are substituted with neutral amino acids. Such a substitution may be, for example, a substitution with an amino acid having a smaller side chain structure, such as alanine.

More preferably, the above-mentioned peptide mutant may be any of the following peptides.
1) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acid residues, and a histidine at the position corresponding to position 10 in the amino acid sequence of SEQ ID NO: 2 or SEQ ID NO: 7 is unsubstituted or substituted with another basic amino acid.
2) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acid residues, and a histidine at the position corresponding to position 10 in the amino acid sequence of SEQ ID NO: 2 or SEQ ID NO: 7 is unsubstituted or substituted with another basic amino acid, and one to several amino acids at positions other than those corresponding to positions 17, 19, 20, 23, 24, and 10 are substituted.
3) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acid residues, and a glutamic acid at the position corresponding to position 6 in the amino acid sequence of SEQ ID NO: 2 or SEQ ID NO: 7 is unsubstituted or substituted with another acidic amino acid.
4) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acid residues, and a glutamic acid at the position corresponding to position 6 in the amino acid sequence of SEQ ID NO: 2 or SEQ ID NO: 7 is unsubstituted or substituted with another acidic amino acid, and one to several amino acids at positions other than those corresponding to positions 17, 19, 20, 23, 24, and 6 are substituted.
5) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acid residues, and a histidine at the position corresponding to position 11 in the amino acid sequence of SEQ ID NO: 2 or SEQ ID NO: 7 is unsubstituted or substituted with another basic amino acid.
6) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 11 in the amino acid sequence of SEQ ID NO: 2 or SEQ ID NO: 7 is unsubstituted or substituted with another basic amino acid, and one to several amino acids at positions other than those corresponding to positions 17, 19, 20, 23, 24, and 11 are substituted.
7) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, and a glutamic acid at the position corresponding to position 6 is unsubstituted or substituted with another acidic amino acid.
8) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, a glutamic acid at the position corresponding to position 6 is unsubstituted or substituted with another acidic amino acid, and one to several amino acids at positions other than those corresponding to positions 17, 19, 20, 23, 24, 10, and 6 are substituted.
9) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, and a histidine at the position corresponding to position 11 is unsubstituted or substituted with another basic amino acid.
10) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, a histidine at the position corresponding to position 11 is unsubstituted or substituted with another basic amino acid, and one to several amino acids at positions other than those corresponding to positions 17, 19, 20, 23, 24, 10, and 11 are substituted.
11) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a glutamic acid at the position corresponding to position 6 in the amino acid sequence of SEQ ID NO: 2 or SEQ ID NO: 7 is unsubstituted or substituted with another acidic amino acid, and a histidine at the position corresponding to position 11 is unsubstituted or substituted with another basic amino acid.
12) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a glutamic acid at the position corresponding to position 6 in the amino acid sequence of SEQ ID NO: 2 or SEQ ID NO: 7 is unsubstituted or substituted with another acidic amino acid, a histidine at the position corresponding to position 11 is unsubstituted or substituted with another basic amino acid, and one to several amino acids at positions other than those corresponding to positions 17, 19, 20, 23, 24, 6 and 11 are substituted.
13) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, a glutamic acid at the position corresponding to position 6 is unsubstituted or substituted with another acidic amino acid, and a histidine at the position corresponding to position 11 is unsubstituted or substituted with another basic amino acid.
14) A peptide containing an amino acid sequence in which one or more amino acid residues selected from the group consisting of amino acid residues corresponding to positions 17, 19, 20, 23, and 24 in the amino acid sequence of SEQ ID NO: 2 or SEQ ID NO: 7 are substituted with any of neutral amino acids, a histidine at the position corresponding to position 10 in the amino acid sequence of SEQ ID NO: 2 or SEQ ID NO: 7 is unsubstituted or substituted with another basic amino acid, a glutamic acid at the position corresponding to position 6 is unsubstituted or substituted with another acidic amino acid, a histidine at the position corresponding to position 11 is unsubstituted or substituted with another basic amino acid, and one to several amino acids at positions other than those corresponding to positions 17, 19, 20, 23, 24, 10, 6, and 11 are substituted.

In an additional aspect of the present application, nucleic acids encoding the above-mentioned peptides and peptide mutants (hereinafter, these are collectively referred to as the "insecticidal peptide of the present application") are provided. Examples of the nucleic acid encoding the insecticidal peptide of the present application include a nucleic acid encoding an insecticidal peptide having an amino acid sequence of SEQ ID NO: 2, SEQ ID NO: 5, or SEQ ID NO: 7, a nucleic acid encoding an insecticidal peptide having an amino acid sequence containing one to several amino acid mutations in the amino acid sequence of SEQ ID NO: 2, SEQ ID NO: 5, or SEQ ID NO: 7, and a nucleic acid encoding an insecticidal peptide including an amino acid sequence having, for example, at least 60%, 65%, 70%, 75%, 80%, 85%, or 90%, preferably at least 95%, more preferably at least 96%, 97%, 98%, or 99% sequence identity to the amino acid sequence of SEQ ID NO: 2, SEQ ID NO: 5, or SEQ ID NO: 7. Additional examples of the nucleic acid encoding the insecticidal peptide of the present application include a nucleic acid containing the nucleotide sequence of SEQ ID NO: 12, SEQ ID NO: 13, or SEQ ID NO: 14, a nucleic acid containing a nucleotide sequence containing, for example, 1 to 30, 1 to 25, 1 to 20, 1 to 15, or 1 to 10, preferably 1 to several nucleotide mutations in the nucleotide sequence of SEQ ID NO: 12, SEQ ID NO: 13, or SEQ ID NO: 14, and a nucleic acid containing a nucleotide sequence having, for example, at least 60%, 65%, 70%, 75%, 80%, 85%, or 90%, preferably at least 95%, and more preferably at least 96%, 97%, 98%, or 99% sequence identity to the nucleotide sequence of SEQ ID NO: 12, SEQ ID NO: 13, or SEQ ID NO: 14.

In the present specification, the term "nucleotide mutation" encompasses substitution, deletion, insertion, and addition of nucleotides. The nucleotide mutation is preferably a silent mutation which does not cause any changes in an encoded amino acid, a nucleotide mutation which causes a conservative amino acid substitution, or a mutation which causes the above-mentioned peptide mutant. Particularly preferably, codons in SEQ ID NO: 12 or SEQ ID NO: 14 corresponding to the amino acids at positions 10, 6, and/or 11 in the amino acid sequence of SEQ ID NO: 2 or SEQ ID NO: 7 may not be mutated, may contain silent mutations, or may be mutated to cause conservative amino acid substitutions. Also preferably, codons in SEQ ID NO: 12 or SEQ ID NO: 14 corresponding to the amino acids at one or more positions selected from the group consisting of positions 17, 19, 20, 23, and 24 in the amino acid sequence of SEQ ID NO: 2 or SEQ ID NO: 7 may be mutated, preferably to cause a substitution with any of neutral amino acids. The nucleotide mutation in the nucleotide sequence encoding the insecticidal peptide of the present application is a mutation that does not affect the insecticidal activity of the encoded peptide, that retains the insecticidal activity of the encoded peptide, or that increases the insecticidal activity of the encoded peptide. A nucleic acid containing nucleotide mutations in the above nucleotide sequence is also encompassed in the nucleic acid encoding the insecticidal peptide of the present application.

The nucleic acid encoding the insecticidal peptide of the present application may be single-stranded or double-stranded DNA or RNA.

In the present specification, the identity of amino acid sequences or nucleotide sequences is determined by aligning a reference sequence and a target sequence for maximum identity and comparing them. The sequence identity (%) can be determined by methods known in the art, for example, using known software such as BLAST, FASTA, SSEARCH, HMMER, DIAMOND, GHOSTZ, or RAPsearch2.

The insecticidal peptide of the present application may be amidated at the C-terminus thereof.

The insecticidal peptide of the present application is preferably an isolated peptide. The nucleic acid encoding the insecticidal peptide of the present application is preferably an isolated nucleic acid. In the present specification, the term "isolated" refers to a state in which a peptide or a nucleic acid is not present in its natural environment, for example, a state in which a peptide or a nucleic acid is separated from the organism from which the peptide or the nucleic acid naturally originates. Therefore, a peptide or nucleic acid is also considered to be "isolated" when the peptide or nucleic acid exists in an organism other than the organism from which the peptide or nucleic acid naturally originates, for example, by genetic engineering technology or the like. For example, the isolated peptides encompass peptides extracted from organisms, recombinant peptides present in host cells such as microorganisms or plant cells, and chemically synthesized peptides. For example, the isolated nucleic acids encompass nucleic acids extracted from organisms, recombinant nucleic acids present in host cells such as microorganisms or plant cells, and chemically synthesized nucleic acids.

The insecticidal peptide of the present application can be produced, for example, by chemical synthesis based on its amino acid sequence, or by genetic engineering using microorganisms or the like based on its nucleotide sequence. Chemical synthesis of the peptide may be carried out by known methods, for example, solid-phase synthesis using a Boc or Fmoc group. The production of peptides by genetic engineering is well known in the art, and the peptides can be produced using known methods. A nucleic acid encoding the insecticidal peptide of the present application can also be produced, for example, by chemical synthesis or by genetic engineering technology using microorganisms or the like, and such methods are well known in the art.

The insecticidal activity of a peptide can be evaluated by administering the peptide to insects and determining the mortality rate or paralysis induction rate after a certain period of time. In the present specification, the term "mortality rate" refers to the ratio of the number of dead individuals relative to the total number of individuals administered with the peptide. In the present specification, the term "paralysis induction rate" refers to the ratio of the number of individuals in which paralysis is induced relative to the total number of individuals administered with the peptide. The "paralysis" can be confirmed by visually observing symptoms such as convulsions or abnormal gait in individuals. Examples of administration methods include oral administration, injection, topical application, and immersion in a drug solution, and the administration method may be selected appropriately depending on the subjects to be administered. The above-mentioned certain period of time is not particularly limited as long as it is a period sufficient for the peptide to take effect in the subjects to be administered. It may be selected appropriately depending on the type or size of the subjects to be administered, the dose of the peptide, or the like. It may be, for example, 60 seconds to 48 hours, preferably 10 minutes to 48 hours, and more preferably 2 hours to 24 hours after administration of the peptide. For example, the insecticidal activity of the peptide may be evaluated by injecting the peptide into the thorax of an insect and determining the mortality rate or paralysis induction rate 48 hours later.

The insecticidal peptide of the present application exhibits an insecticidal effect against any common insect pests. Examples of the insect pests include, but are not limited to, insects of the orders Orthoptera, Blattaria, Tetranychidae, and Hemiptera, such as crickets, grasshoppers, cockroaches, termites, spider mites, and aphids. The insecticidal peptide of the present application is not toxic to mammals such as mice, dogs, cats, and humans, and exhibits an insecticidal effect specific to insects. In addition, insects administered with the insecticidal peptide of the present application are characterized by exhibiting convulsive symptoms. In other words, the insecticidal peptide of the present application induces paralysis in the insect, and then leads to its death. It is also possible to induce only paralysis without killing the insect by appropriately selecting the type or dosage of the peptide.

### 2. Insecticidal composition

In an additional aspect of the present application, an insecticidal composition containing the insecticidal peptide of the present application is provided. The insecticidal composition of the present application can be used as an agrochemical, insecticide, or the like, to control the above-mentioned insect pests.

The insecticidal composition of the present application may be the insecticidal peptide of the present application itself, or may contain, in addition to the insecticidal peptide of the present application, an inert carrier, and, as needed, an additive such as a surfactant or a formulation adjuvant. The insecticidal composition of the present application can be formulated by a conventional method, and may be formulated, for example, into an oil agent, emulsion, liquid agent, flowable agent, water-soluble agent, wettable powder, water dispersible granule, powder agent, granule, powdered granule, microcapsule, aerosol, or the like.

Although the amount of the insecticidal peptide of the present application in the insecticidal composition of the present application is not particularly limited, the amount may be, for example, 0.01 to 90% by weight, preferably 0.1 to 80% by weight.

Examples of the inert carrier used in formulation include solid carriers, liquid carriers, and gaseous carriers, and carriers known in the art can be used. Examples of the solid carriers include, but are not limited to, fine powders and granules of minerals such as kaolin clay, attapulgite clay, talc, and diatomaceous earth; natural organic substances such as corncob flour, peanut shell flour, and bran; synthetic organic substances such as urea and urea formaldehyde resin; salts such as calcium carbonate and ammonium sulfate; and synthetic inorganic substances such as synthetic hydrated silicon oxide. Examples of the liquid carriers include, but are not limited to, water; aromatic hydrocarbons such as xylene, alkylbenzenes, and methylnaphthalene; alcohols such as 2-propanol, ethylene glycol, propylene glycol, and ethylene glycol monoethyl ether; ketones such as acetone, cyclohexanone, and isophorone; vegetable oils such as soybean oil and cottonseed oil; petroleum-based aliphatic hydrocarbons; esters; dimethyl sulfoxide; and acetonitrile. Examples of gaseous carriers include, but are not limited to, fluorocarbons, butane gas, LPG (liquefied petroleum gas), dimethyl ether, and carbon dioxide.

Examples of the surfactant include, but are not limited to, anionic surfactants such as alkyl sulfate ester salts, alkylaryl sulfonates, dialkyl sulfosuccinates, polyoxyethylene alkylaryl ether phosphate ester salts, lignin sulfonates, naphthalenesulfonate formaldehyde polycondensates, styrene-acrylic acid copolymers, and sodium methyloleyl taurate salts; nonionic surfactants such as polyoxyethylene alkylaryl ethers and sorbitan fatty acid esters; and cationic surfactants such as alkyltrimethylammonium salts. Examples of other formulation adjuvants include, but are not limited to, water-soluble polymers such as polyvinyl alcohol and polyvinylpyrrolidone; polysaccharides such as gum arabic, alginic acid and salts thereof, carboxymethyl cellulose, and xanthan gum; inorganic substances such as aluminum-magnesium silicate and alumina sol; preservatives such as 5-chloro-2-methyl-4-isothiazolin-3-one, 1,2-benzothiazolin-3-one, and 2-bromo-2-nitropropane-1,3-diol; colorants; and stabilizers such as PAP (acidic isopropyl phosphate) and BHT (2,6-di-tert-butyl-4-methylphenol).

### 3. Method for controlling insect pests

In an additional aspect of the present application, a method for controlling insect pests, including applying the insecticidal peptide of the present application to insect pests or habitats of the insect pests is provided. In the method, the insecticidal composition of the present application is preferably applied as the insecticidal peptide of the present application. Examples of the habitats of insect pests include, but are not limited to, crops and soil in which crops grow, wooden houses, and indoor spaces such as kitchens, washrooms, and bathrooms. Examples of the crops include, but are not limited to, rice, cabbage, Chinese cabbage, rapeseed, perilla, mint, soybean, and garland chrysanthemum.

Although the method for applying the insecticidal peptide of the present application to insect pests or habitats of the insect pests may be appropriately selected by those skilled in the art and is not particularly limited, examples thereof include spraying the insecticidal peptide of the present application on insect pests, mixing the insecticidal peptide of the present application with bait to feed the mixture to insect pests, scattering the insecticidal peptide of the present application on the stems and leaves of crops, scattering the insecticidal peptide of the present application on the soil or irrigating the soil with the insecticidal peptide of the present application, and treating seeds of crops with the insecticidal peptide of the present application. Furthermore, insect pests can be controlled by producing a plant resistant to insect pests by expressing the insecticidal peptide of the present application in a plant by gene-recombination. Furthermore, insect pests can be eliminated by incorporating the insecticidal peptide of the present application into a virus that infects only insects, such as baculovirus, to infect the insect pests, thereby expressing the insecticidal peptide of the present application in the bodies of the insect pests.

The application amount of the insecticidal peptide in the method for controlling insect pests of the present application is not particularly limited, and can be appropriately selected depending on the insect pests to be applied, habitats of the insect pests, the application method, or the like.

Although the present invention will be explained in more detail below with reference to examples, the present invention is not limited to these examples.

### Examples

### Example 1. Identification of sequence of candidate peptides

Total RNA was extracted from the telson of *L. australasiae* scorpion, and a cDNA library was prepared using NEBNext Ultra RNA Library Prep Kit for Illumina. The resulting library was subjected to a 100bp paired-end sequence-analysis using HiSeq 2500 (Illumina) to obtain DNA sequence fragments (reads). An open reading frame (ORF) was extracted from the contig sequence obtained by assembling the read sequence. The open reading frame was subjected to a BLAST search to obtain sequences similar to those of known peptides derived from scorpion venom. The presence or absence of a signal sequence, a propeptide region, and a C-terminal amidation modification were estimated from the obtained sequences, and the mature sequences of La-κKTx1 (2592.0 Da), La-κKTx2 (2687.0 Da), La-xKTx3 (2745.4 Da), La-κKTx4 (2857.4 Da), and La-κKTx5 (2639.2 Da) were obtained. The sequence of each peptide is shown in Table 1.

**[Table 1]**

| Peptide | Amino acid sequence (* indicates that C-terminus is amidated.) |
|---|---|
| La- 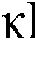 KTx1 | GNSCMEVCLQHEGNVAECEKACNK (SEQ ID NO: 1)* |
| La- 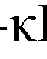 KTx2 | DSACVEVCLHHEGNVAECEEACKKS (SEQ ID NO: 2) |
| La- 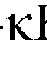 KTx3 | LVQKPCRIVCSENMRKCIRRCTL (SEQ ID NO: 3)* |
| La- 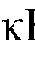 KTx4 | AERAGCRIRCLQFTDDFEKCRKLC (SEQ ID NO: 4)* |
| La- 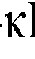 KTx5 | SCKRVCSGTRRTKKCMQKCKSQP (SEQ ID NO: 5)* |

### Example 2. Synthesis of candidate peptides

### (1) Reagents

Fmoc amino acids (Table 2), peptide synthesis resins C1-MPA ProTide (LL), RINK AMIDE ProTide (LL), and Oxyma Pure were purchased from CEM. Piperidine, N,N'-dimethylformamide (DMF), diethyl ether, dichloromethane (DCM), N,N'-diisopropylethylamine (DIPEA), trifluoroacetic acid (TFA), reduced glutathione, oxidized glutathione, tris(hydroxymethyl)aminomethane, CH₃CN (HPLC grade), formic acid (HPLC grade), and distilled water (HPLC grade) were purchased from NACALAI TESQUE, Inc. N,N'-diisopropylcarbodiimide (DIC) was purchased from FUJIFILM Wako Pure Chemical Corporation. 1,2-Ethanediol (EDT) was purchased from Sigma-Aldrich Co. LLC. Triisopropylsilane (TIS) was purchased from Tokyo Chemical Industry Co., Ltd. Other commercially available reagents were used.

**[Table 2]**

| Table 2 Used Fmoc amino acids | | | |
|---|---|---|---|
| Fmoc-Ala-OH | Fmoc-Cys(Trt)-OH | Fmoo-Asp(OtBu)-OH | Fmoc-Glu(OtBu)-OH |
| Fmoc-Gly-OH | Fmoc-His(Trt)-OH | Fmoc-Lys(Boc)-OH | Fmoc-Leu-OH |
| Fmoc-Asn(Trt)-OH | Fmoc-Pro-OH | Fmoc-Gln(Trt)-OH | Fmoc-Ser(tBu)-OH |
| Fmoc-Val-OH | Fmoc-Tyr(iBu)-OH | | |

### (2) Synthesis method

Based on each amino acid sequence obtained in Example 1, each candidate peptide was synthesized by Fmoc solid-phase synthesis. Introduction of the first amino acid to resin, deprotection of Fmoc group, and elongation of peptide chain were all carried out using an automatic microwave peptide synthesizer under the trade name of Liberty Lite (CEM). The introduction and elongation reaction conditions are described below.

### Introduction condition of the first amino acid to resin

Reaction reagents: KI (2.5 equivalents), DIPEA (20 equivalents), Fmoc amino acid (10 equivalents)
Reaction time: 80°C for 60 seconds, 90°C for 540 seconds

### Elongation reaction condition of amino acid

Deprotection
   Reaction reagent: 20% Piperidine/DMF
   Reaction time: 75°C for 15 seconds, 90°C for 50 seconds
Coupling reaction of Fmoc amino acid
   (i) Fmoc amino acid other than histidine
      Reaction reagents: Oxyma Pure (5 equivalents), DIPEA (0.5 equivalents), DIC (10 equivalents), amino acid (5 equivalents)
      Reaction time: 75°C for 15 seconds, 90°C for 110 seconds
   (ii) Histidine
      Reaction reagents: Oxyma Pure (5 equivalents), DIPEA (0.5 equivalents), DIC (10 equivalents), amino acid (5 equivalents)
      Reaction time: 25°C for 120 seconds, 50°C for 240 seconds

### Cleavage of peptide from resin

The resin after the completion of the elongation of peptide chain was washed five times each with DMF, DCM, and ether, and then dried under reduced pressure overnight in a desiccator. A cleavage solution (94% TFA, 2.5% H₂O, 2.5% EDT, and 1.0% TIS) was added at a ratio of 20 mL per 1 g of dried resin, and the mixture was stirred at room temperature for 120 minutes. After the reaction was completed, the reaction mixture was filtered by suction through a glass filter, and the filtrate was poured directly into ice-cold ether in an amount 10 times the reaction mixture to precipitate the peptide. After this mixture was stirred well, it was centrifuged at 2500 rpm for 10 minutes, and the supernatant was removed. To the resulting precipitate, ice-cold ether in an amount 10 times the reaction mixture was added, and it was stirred and centrifuged in the same manner. Ether was added to the precipitate once more, the mixture was stirred and centrifuged, and the resulting peptide was dried under reduced pressure overnight in a desiccator.

### Disulfide-bond cross-linking reaction and purification

The resultant peptide was dissolved in a buffer (1 mM reduced glutathione, 0.1 mM oxidized glutathione, 0.2 M Tris-HCl, pH 8.0) such that the concentration of the resultant peptide became 1.0 mg/mL, and then allowed to stand still at room temperature for 24 hours. After completion of the reaction, purification was carried out by reverse phase HPLC under the following conditions.
Column: Hydrosphere C18 250 × 20 mm (YMC)
Column temperature: 40°C
Solvent: (A) 0.1% TFA/H₂O, (B) 0.1% TFA/CH₃CN
Gradient: 5-50% (B) 30 minutes
Flow rate: 7.0 mL/min
UV detection: 215 nm

It was confirmed by LC/MS analysis under the following conditions that the collected fraction contained the target peptide.
Mass spectrometer: LCMS-2020 (SHIMADZU)
Column: DAISOPAK 2.0 × 150 mm (OSAKA SODA)
Column temperature: 40°C
Solvents: (A) 0.1% formic acid/H₂O, (B) 0.1% formic acid/CH₃CN
Gradient: 5-50% (B) 20 minutes
Flow rate: 0.2 mL/min
Ionization method: Electrospray ionization (ESI), positive mode

CH₃CN contained in the fraction was removed by a centrifugal evaporator, and then the resultant aqueous solution was lyophilized. The final purity of the peptide was confirmed to be 95% or higher by LC/MS analysis.

### Example 3. Evaluation of insecticidal activity of candidate peptides

A solution of each candidate peptide synthesized in Example 2 was injected into the thorax of ice-cold anesthetized crickets (*Acheta domesticus,* 45 to 55 mg) (n=10). The number of individuals with paralysis induced after 1 hour and the number of dead individuals after 48 hours were counted. Paralysis was judged based on the presence or absence of convulsions or gait abnormalities. The results are shown in Table 3. Furthermore, La-κKTx2 was evaluated in the same manner using cockroaches (*Periplaneta americana,* 100 to 400 mg) (n=3).

**[Table 3]**

| Peptide | Dose (µg/g body weight) | Number of individuals with paralysis | Number of dead individuals |
|---|---|---|---|
| La-κKTx1 | 200 | 0 / 10 (crickets) | 0 / 10 (crickets) |
| La-κKTx2 | 200 | 10 / 10 (crickets) | 10 / 10 (crickets) |
| La-κKTx3 | 200 | 0 / 10 (crickets) | 0 / 10 (crickets) |
| La-κKTx4 | 200 | 1 / 10 (crickets) | 0 / 10 (crickets) |
| La-κKTx5 | 200 | 3 / 10 (crickets) | 1 / 10 (crickets) |

As is clear from Table 3, among the candidate peptides, La-κKTx2 and La-κKTx5 exhibited insecticidal activity against crickets. In particular, La-κKTx2 exhibited strong insecticidal activity. La-κKTx5 induced paralysis, but did not lead some individuals to death. The individuals showed convulsive symptoms in which the thorax, where the peptide was injected, contracted no later than 30 minutes after administration of La-κKTx2 or La-κKTx5, and then died. In the La-κKTx5 administration group, some individuals were paralyzed but did not die. Furthermore, evaluation of the insecticidal activity against cockroaches showed that administration of 6.25 µg/g body weight induced severe paralysis in all individuals.

### Example 4. Modification of peptide sequence and evaluation of insecticidal activity

La-κKTx2, La-xKTx1, and OmTx1 (SEQ ID NO: 6), which is a known κ-KTx peptide derived from *Opisthacanthus madagascariensis* and has an amino acid sequence similar to that of La-κKTx2, were evaluated in terms of an insecticidal activity, and the median lethal dose (LD₅₀) of each peptide was determined. The evaluation test in terms of an insecticidal activity was carried out on crickets by the same method as that of Example 3, except that the dose of the peptide was 200 µg/g body weight (n=10). As a result, it became clear that OmTx1 did not exhibit insecticidal activity in a similar manner to La-κKTx1. The results are shown in Table 4.

**[Table 4]**

| Peptide | LD₅₀ (µg / g body weight) |
|---|---|
| La-κKTx2 | 28 |
| La-κKTx1 | >200 |
| OmTx1 | >200 |

Next, the amino acid sequences of these three peptides were aligned and compared, and it was noted that the basic amino acid histidine was present only at position 10 in the amino acid sequence of La-κKTx2 (Table 5). The positions corresponding thereto in La-κKTx1 and OmTx1 are positions 10 and 9, respectively, and both amino acid residues at these positions are glutamine. In order to examine whether the basic amino acid residue at this position is involved in the insecticidal activity, a peptide "La-κKTx2(H10Q)" (SEQ ID NO: 8) in which the amino acid residue at position 10 in the amino acid sequence of La-κKTx2 was substituted with glutamine, and a peptide "La-xKTx1(Q10H)" (SEQ ID NO: 7) in which the glutamine at position 10 of the amino acid sequence of La-κKTx1 was substituted with histidine were synthesized (Table 5), and their insecticidal activity was evaluated in the same manner as above. In addition, peptides "La-κKTx2(E6A)" (SEQ ID NO: 9) and "La-xKTx2(E12A)" (SEQ ID NO: 10) in which glutamic acid at position 6 or position 12 in the amino acid sequence of La-κKTx2, which was predicted to be involved in the insecticidal activity from a comparison with La-κKTx1, was substituted with alanine, a peptide "La-κKTx2(E20K)" (SEQ ID NO: 11) in which glutamic acid at position 20 in the amino acid sequence of La-κKTx2 was substituted with lysine, and a peptide "La-κKTx2(amide)" in which the C-terminal structure of La-κKTx2 was converted to an amide group were synthesized (Table 5), and the insecticidal activity thereof was evaluated in the same manner as above. The results are shown in Table 6.

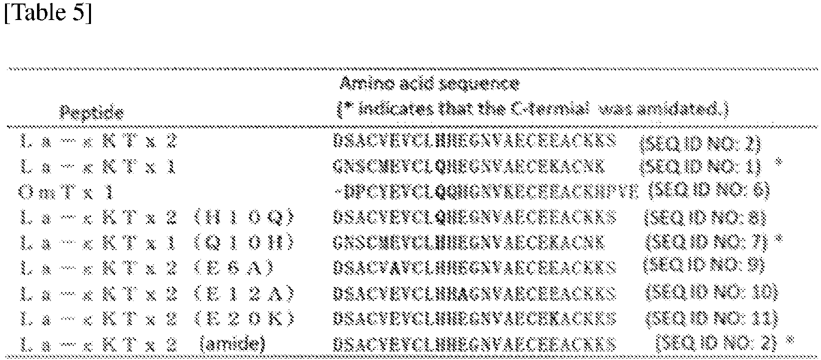

**[Table 6]**

| Peptide | LD₅₀ (µg / g body weight) |
|---|---|
| La-κKTx2 | 28 |
| La-κKTx2(H10Q) | 142 |
| La-κKTx1 | >200 |
| La-κKTx1(Q10H) | 28 |
| La-κKTx2(E6A) | 56 |
| La-κKTx2(E12A) | 26 |
| La-κKTx2(E20K) | 32 |
| La-κKTx2(amide) | 20 |

As a result, it became clear that the insecticidal activity of La-κKTx2 was reduced to about one-fifth when histidine at position 10 in La-κKTx2 was substituted with glutamine. On the other hand, it became surprisingly clear that the substitution at position 10 in the amino acid sequence of La-κKTx1, which does not exhibit insecticidal activity, with histidine made it possible to exhibit insecticidal activity equivalent to that of La-κKTx2. Furthermore, a peptide in which glutamic acid at position 6 of La-κKTx2 was substituted with alanine exhibited about half the activity of the original peptide, but the substitution of glutamic acid at position 12 with alanine and the substitution of glutamic acid at position 20 with lysine had no effect on the activity. Therefore, it became clear that in the insecticidal peptide of the present application, a histidine residue at position 10 in the amino acid sequence of La-κKTx2 is most important for its insecticidal activity, and a glutamic acid residue at position 6 is also involved in the activity to some extent. On the other hand, when a C-terminal carboxyl group of La-κKTx2 was substituted with an amide group, a slight increase in insecticidal activity was observed. Thus, it became clear that the C-terminal structure of La-κKTx2 is also involved in the activity. However, since the difference in activity was slight, it was found that the insecticidal peptide of the present application exhibits sufficient insecticidal activity even when the C-terminus is not amidated. The three-dimensional structure of La-κKTx2 is shown in Fig. 1. As shown in Fig. 1, the histidine at position 10 and the glutamic acid at position 6, which were found to be involved in the activity, were present on the same face of a single α-helix structure, and therefore it was assumed that they interact with the target molecule on this face.

### Example 5. Evaluation of toxicity to mammals

In order to examine whether the insecticidal peptide of the present application exhibits toxicity to mammals, La-κKTx2 was administered to mice to determine the median lethal dose (LD₅₀). Specifically, La-κKTx2 was administered intracerebroventricularly to 4-week-old mice (male, 20 g) at 200 µg/kg body weight, and the LD₅₀ was calculated from the mortality rate one day later. The results are shown in Table 7. As a control, the literature values of the scorpion venom peptides AaH I, AaH II, AaH III, TsTx, Tsy, and Css II, which are known to exhibit toxicity to mammals, are shown (Schweitz, Toxicon, 22, 308, 1983).

**[Table 7]**

| Peptide | LD₅₀ (µg/kg body weight) |
|---|---|
| La-κKTx2 | >200 |
| AaH I | 0.7 |
| AaH II | 12 |
| AaH III | 2.7 |
| TsTx | 1.7 |
| Tsγ | 37 |
| Css II | 1.6 |

It became clear that the insecticidal peptide of the present application was not toxic to mice, and exhibited action specific to insects.

### Examples of nucleotide sequence encoding peptide having insecticidal activity of the present application

La-κKTx2: 
GATTCGGCTTGTGTGGAAGTGTGCCTTCATCATGAAGGAAATGTAGCAGAGTG

TGAAGAAGCATGCAAAAAGAGC (SEQ ID NO: 12)
La-κKTx5: 
TCGTGCAAAAGAGTGTGCTCGGGGACCAGACGCACGAAAAAATGCATGCAG
AAGTGTAAATCACAGCCT (SEQ ID NO: 13)
La-κKTx1(Q10H):

GGTAATTCCTGTATGGAAGTGTGCCTTCATCATGAAGGAAATGTAGCAGAGTG
TGAAAAAGCATGCAATAAG (SEQ ID NO: 14)

### Example 6. Modification of peptide sequence and evaluation of insecticidal activity

According to the same method as that described in Example 4, each of the basic amino acid residues and acidic amino acid residues (namely, at positions 1, 11, 17, 19, 20, 23 and 24) in the amino acid sequence of the La-κKTx2 peptide was substituted with alanine (namely, neutral amino acid) to synthesize a modified peptide, and its insecticidal activity was evaluated. The amino acid sequences and insecticidal activities of the synthesized modified peptides are shown in Tables 8 and 9.

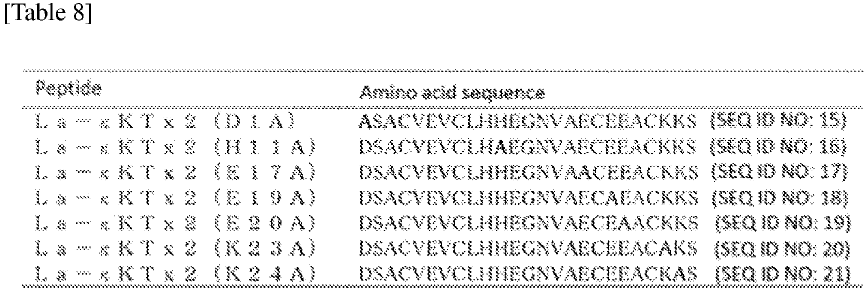

**[Table 9]**

| Peptide | LD₅₀ (µg/g body weight) |
|---|---|
| La-κKTx2(D1A) | 37 |
| La-κKTx2(H11A) | 131 |
| La-κKTx2(E17A) | 11 |
| La-κKTx2(E19A) | 14 |
| La-κKTx2(E20A) | 9.6 |
| La-κKTx2(K23A) | 13 |
| La-κKTx2(K24A) | 8.8 |

As a result, the insecticidal activity of the peptide "La-κKTx2(H11A)" in which histidine at position 11 was substituted with alanine was significantly reduced compared to the insecticidal activity of the La-κKTx2 peptide (SEQ ID NO: 2) free from sequence modification (LD₅₀ value = 28 µg/g body weight; see Example 4). Therefore, it became clear that in the insecticidal peptide of the present application, the presence of a basic amino acid (such as histidine) at position 11 is also involved in the insecticidal activity in a similar manner to the presence of a basic amino acid (such as histidine) at position 10 in the amino acid sequence of La-κKTx2. Since histidine at position 10 and glutamic acid at position 6, and further histidine at position 11, which were found to be involved in the activity, are present outside the same single α-helix structure, as shown in FIG. 1, it was assumed that they interact with the target molecule at this helix structure portion.

Furthermore, it became clear that the substitution of glutamic acid or lysine at positions 17, 19, 20, 23, and 24 of La-κKTx2 with alanine surprisingly made it possible to exhibit insecticidal activity more than twice that of La-κKTx2. On the other hand, when aspartic acid at position 1 and glutamic acid at position 12 (Example 4) were substituted with alanine, such a significant increase in insecticidal activity was not observed. As shown in FIG. 2, the amino acid positions of positions 17, 19, 20, 23, and 24 in La-κKTx2 are all located outside the same single α-helix structure. Therefore, it became clear that the insecticidal activity was increased by substituting the amino acid residues located at the above-mentioned positions in the C-terminal side of α-helix (in the amino acid sequence of half of the C-terminal side in the amino acid sequence of La-κKTx2), namely, located outside the α-helix structure at the C-terminal side, of two α-helices that constitute La-κKTx2.

## Claims

1. A peptide having an insecticidal activity, the peptide comprising an amino acid sequence of SEQ ID NO: 2, SEQ ID NO: 5, or SEQ ID NO: 7, or an amino acid sequence having one to several amino acid mutations in the amino acid sequence of SEQ ID NO: 2, SEQ ID NO: 5, or SEQ ID NO: 7.

2. The peptide according to claim 1, wherein an amino acid residue corresponding to a position 10 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is a basic amino acid.

3. The peptide according to claim 1 or 2, wherein an amino acid residue corresponding to a position 6 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is an acidic amino acid.

4. The peptide according to any one of claims 1 to 3, wherein an amino acid residue corresponding to a position 11 in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is a basic amino acid.

5. The peptide according to any one of claims 1 to 4, wherein at least one amino acid residue corresponding to at least one position of positions 13 or later in the amino acid sequence of SEQ ID NO: 2 or SEQ ID NO: 7 in the amino acid sequence having one to several amino acid mutations in the amino acid sequence of SEQ ID NO: 2 or SEQ ID NO: 7 is substituted with another amino acid.

6. The peptide according to claim 5, wherein the at least one amino acid residue corresponding to at least one position of positions 13 or later is at least one amino acid residue selected from the group consisting of amino acid residues corresponding to positions 17, 19, 20, 23 and 24.

7. The peptide according to any one of claims 1 to 6, wherein a C-terminus is amidated.

8. An insecticidal composition comprising the peptide of any one of claims 1 to 7.

9. The insecticidal composition according to claim 8, wherein the insecticidal composition is in a form selected from the group consisting of oil agents, emulsions, liquid agents, flowable agents, water-soluble agents, wettable powders, water dispersible granules, powders, granules, powdered granules, microcapsules and aerosols.

10. A method for controlling insect pests, comprising applying the peptide of any one of claims 1 to 7 to the insect pests or a habitat of the insect pests.

11. A nucleic acid encoding the peptide of any one of claims 1 to 6.
